# EUROPEAN PATENT SPECIFICATION

(11) **EP 4 161 613 B1**
(45) Date of publication and mention of the grant of the patent: **06.03.2024**
(21) Application number: 21734476.1
(22) Date of filing: 03.06.2021
(51) Int. Cl.: A61M 15/00, A61M 15/06, A24F 15/015, B65D 83/42

(54) **A REFILL FOR AN INHALER PARTICULARLY A CANNABINOID INHALER**
NACHFÜLLUNG FÜR EINEN INHALATOR, INSBESONDERE EINEN CANNABINOIDINHALATOR
RECHARGE POUR UN INHALATEUR, EN PARTICULIER UN INHALATEUR DE CANNABINOÏDES

(30) Priority: 03.06.2020 GB 202008359
(43) Date of publication of application: 12.04.2023
(73) Proprietor: OBG Consumer Reg Scientific Limited, Liverpool L8 7BA (GB)
(72) Inventor: YOUNG, Paul, Liverpool L8 7BA (GB)
(74) Representative: Boult Wade Tennant LLP
(86) International application number: PCT/GB2021/051378
(87) International publication number: WO 2021/245417

(56) References cited:
- WO-A1-2010/073018
- WO-A1-2014/155092
- WO-A1-2019/073204
- GB-A- 1 154 268
- GB-A- 2 542 404
- GB-A- 2 549 769

## Description

The present invention relates to a refill for an inhaler, particularly a cannabinoid inhaler.

Cannabinoids have long been known for their therapeutic potential in pain relief, treatment of seizures, antiemesis et cetera. It is, however, a class of compounds whose usage has been associated with a great deal of debate owing to its psychoactive effects. It was not until the discovery of cannabinoid receptors (CB1 and CB2) and the isolation of individual cannabinoids such as THC (tetrahydrocannabinol), CBD (cannabidiol), CBN (Cannabinol), and THCV (Tetrahydrocannabivarin), that the psychoactive effects could be attributed primarily to compounds (like THC) with high affinities to the receptor CB1. Furthermore, it has been established that individual cannabinoids differ from one another in their affinities to receptors and certain cannabinoids, such as CBD, behave as CB1/CB2 antagonists, thereby blocking some actions of their agonists, such as THC.

With on-going research, therapeutic applications of cannabinoids are becoming increasingly evident, resulting in legalisation of these compounds for medical purposes in a number of countries. The primary targets of research in this field are being associated with safe, rapid and/or effective delivery of cannabinoids.

A number of ways of delivering cannabinoids are known in the art.

For example, US2012/0304990 teaches the use of heating to vaporise a cannabis deposit. One drawback of this system is that there is only a small temperature differential between the temperature at which the cannabis will vaporise (180°c to 200°c) and the temperature at which toxins are produced (230°c).

A number of documents (for example WO03/055549, US6509005 and WO2004/000290) disclose the use of a metered dose inhaler. Such inhalers suffer from a number of drawbacks. Firstly, the metering chamber is relatively small, generally less than 100µl resulting in delivery of fairly concentrated doses. Also, such devices require users to optimally co-ordinate actuation of the outlet valve and inhalation, failing which, dose delivery could be variable.

A further common mechanism is the simple spray which is disclosed, for example, in WO02/064109 and US2006/135599 which are designed to provide a sublingual or buccal spray. Such a spray is currently being marketed by GW Pharmaceuticals under the Sativex (TM) brand. These sprays suffer from the possibility of non-uniform drug dose delivery owing to the flushing action of saliva. Further, they have a slower onset of action when compared with pulmonary delivery.

A development of this idea was disclosed in WO2015/121673. This takes an inhaler which is based on a simulated cigarette and uses it to dispense cannabis.

The inhaler is based on a design of simulated cigarette which uses a pressurised reservoir and a breath operated valve. Suction on an inhaling end of the inhaler opens the breath operated valve such that the pressurised reservoir is able to dispense the formulation.

The inhaler is primarily designed as a simulated cigarette. Details of the breath operated valve are described in WO2009/001082, WO2010/073018, WO2011/015825, WO2011/015826, WO2014/033438, WO2014/033439, WO2014/155091 and WO2016/005728.

Additional details of the inhaler are provided in: WO2011/117580 and WO2014/155093

Details of the manner in which the inhaler is tested and assembled are provided in WO2015/087045, WO2014/155095 and WO2016/046567.

The inhaler is designed to be refillable via a refill pack which is designed to have the shape and size of a cigarette pack. Details of the refill pack are provided in:
WO2009/001078, WO2014/155092, WO2014/155090 and WO2014/155089
WO2015/121673 provides details of the composition required to dispense a cannabinoid based product from such an inhaler.

The above mentioned references provide a full description of the inhaler internal workings and the composition.

A disclosure of an inhaler which has been modified in order to be suitable for dispensing a cannabinoid product is disclosed in WO 2019/073204. This takes the above described inhaler and encases it in an outer housing in order to create a larger inhaler which can more easily be handled by a user. The inhaler is packaged in a refill pack which fulfils a number of functions in that it houses a refill canister which is protected from dirt and damage during transportation and storage, and also provides guidance for the interface between the inhaler and the refill cannister during the refill operation. On the other hand, the refill housing is a relatively large plastic component which is disposable once the contents of the refill device are exhausted and which cannot easily be recycled.

The present invention addresses this concern while maintaining the key functionality of the refill housing of WO 2019/073204.

According to the present invention, there is provided a refill for an inhaler according to claim 1.

Rather than providing a refill housing to fully enclose the refill and the inhaler, the present invention replaces this with a cap fitted onto the end of the refill cylinder. This uses a fraction of the material required for the housing in WO 2019/073204. The fact that the cap extends axially beyond the valve stem provides protection for the valve stem from damage and dirt during transportation and storage. The cap provides the possibility that a film can be applied, for example in the form of a sticker or a shrink fit across the cap in order to provide further protection and to provide a tamper evident feature.

Further, because the refill is a cylinder which is refilled by engagement with a cap on the top of the cylinder, the refill and inhaler are effectively refilled by pressing the two components together in a direction along the central axis of the refill. This provides a more robust refill arrangement as the user can grip the refill in one hand and the inhaler in the other hand and push them together in an end-to-end manner along the axis of the refill. This contrasts with WO 2009/073204 where the axis of the refill device is offset a significant distance from the centre of the refill housing making the refill operation more awkward, particularly for those with a limited dexterity in their hands.

The cap may be bonded to the cylinder. However, preferably, the cap is a snap fit onto the cylinder as this provides a simple and robust attachment. Further, preferably, a sticker surrounds both the cap and the cylinder to provide a more robust connection and to provide a tamper evident feature.

In WO 2019/073204, the refill end of the inhaler and the corresponding guide surface in the refill pack are circular so that the inhaler can be refilled in any angular orientation.

In the present case, the internal passage extending through the cap preferably has a non-circular cross-section. This allows the interface between the refill and the inhaler to be made larger than it is in WO 2019/073204. Further, the refill and inhaler cannot rotate relatively to one another when they are engaged during the refill process. Both of these features, coupled with the end-to-end axial arrangement set out above lead to a much more robust refilling process.

The internal passage preferably has an elongate generally oval shape. This fits well with the overall shape of the inhaler. The internal passage preferably has at least one inwardly protecting axially extending rib which can provide a location feature with a corresponding recess on the refill end of the inhaler. The internal passage may also be tapered to aid in the location of the inhaler in the internal passage.

The cross sectional area of the internal passage in a plane perpendicular to the axial direction is at least 30% and more preferably at least 40% of the cross sectional area of the cap in a plane perpendicular to the axial direction. This provides a large surface for the interface with the inhaler which provides for a robust refill and allows the inhaler to have a relatively large engaging end.

Preferably the valve stem is depressible axially to open the valve. This provides a robust refill operation.

The present invention may also extend to a combination of a refill according to a first aspect of the present invention and an inhaler, the inhaler having a housing with a refill end and an inhaling end, the inhaling end being dimensioned to fit within the internal passage in the refill, and the inhaling end being dimensioned so that it will not fit within the internal passage in the refill. This prevents the user from inadvertently trying to refill the inhaler in the incorrect orientation.

An example of a refill container in accordance with the present invention, and an inhaler which can be used with the refill, will now be described with reference to the accompanying drawings, in which:
Figure 1 is a perspective view from one end of the inhaler from below;
Figure 2 is a perspective view from one end of the inhaler from above;
Figure 3 is a perspective view in the same orientation as Figure 2 of an internal portion of the inhaler;
Figure 4 is a cross-section through a medium plane at the inhaling end of the inhaler;
Figure 5 is a perspective view from above of the refill;
Figure 6 is a top view of the refill;
Figure 7 is perspective view from above of the cap; and
Figure 8 is a perspective view from below of the cap.

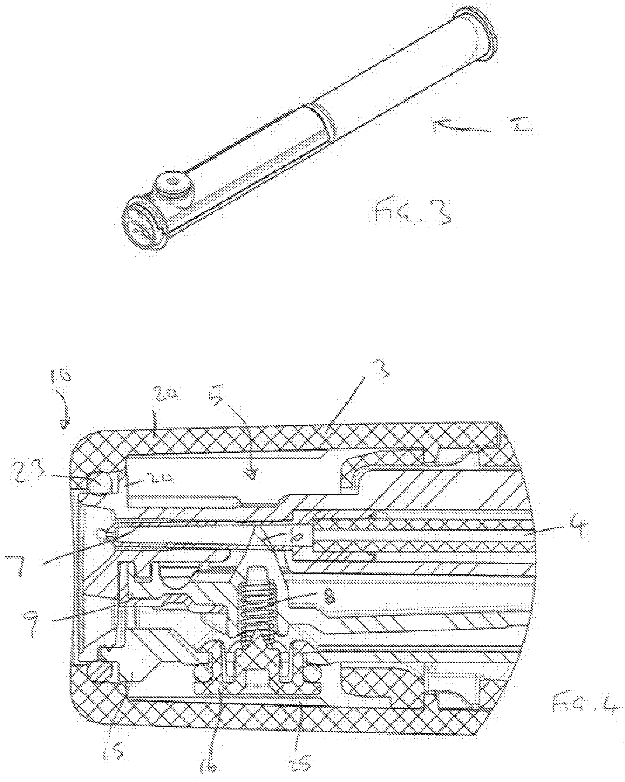

The internal workings of the inhaler device 1 are based on an inhaler core I shown in Fig. 3 which is described in the PCT publications referred to above. This forms the inner core of the inhaler device. This is modified as described in WO2019/073204 to surround this inner core I with an outer housing 3 as described below.

In general terms, the workings of the inhaler core I have not been modified and this will therefore not be described in great detail here.

In broad terms, the inhaler core I comprises a reservoir 4 and a breath operated valve mechanism 5 which has a spring loaded member valve member 6 (see Fig. 4) which pinches a deformable tube 7 closed to close off access to the reservoir. The valve element 6 is biased closed by a spring 8. A membrane 9 is in communication with an air flow path such that suction on the inhaling end 10 (opposite to refill end 14) causes a change of pressure on the membrane which will lift the valve element 6 against the action of the spring 8. Further details of this are given in the above references.

One modification to the inhaler core I is illustrated in Fig. 4. The inhaler I is closed in the vicinity of the membrane 9 by a cap 15 which in the above mentioned references directly support the spring 8. In the present case, however, a further component in the form of a bung 16 is inserted through an opening 17 in the cap and is sealed by an O ring 18. This allows the cap 15 to be welded in place before the spring 8 is put in place and then retained by the bung 16.

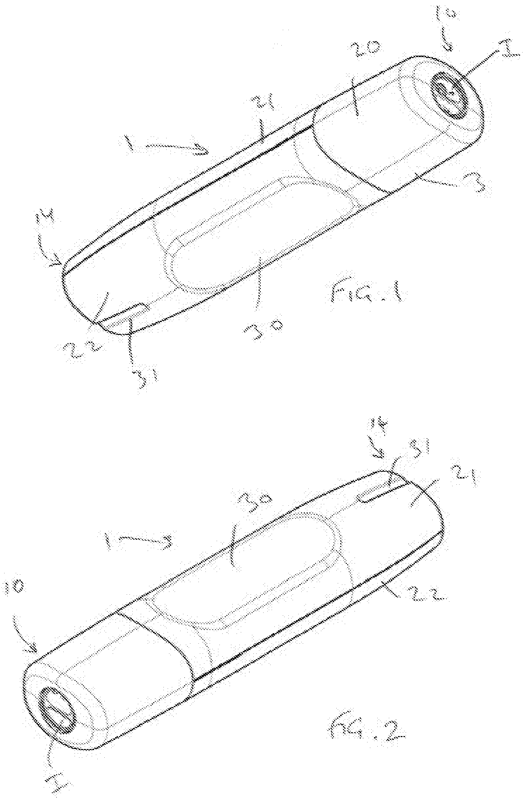

As is apparent, for example, from Figs. 1 and 2, the outer housing 3 is made up of three separate portions, namely a mouthpiece casing 20, a top casing 21 and a bottom casing 22 which are clipped together to form the outer housing. As can be seen in Fig. 4, an O-ring 23 seals a flange 24 on the inhaler core to the mouthpiece casing 20. This flange 24 represents a further modification of the inhaler I. A similar flange is present at the opposite end to the outer housing 3. The mouthpiece casing 20 is also provided with a number of ribs 25 (only one of which is visible in Fig. 4) to prevent the bung 16 from being dislodged.

As shown particularly in Figs. 1 and 2, the outer housing 3 is provided with a number of ergonomic features to enhance the usability of the inhaler device 1 particularly for those with limited manual dexterity.

Thus, the outer housing 3 is provided with a significantly larger size than the inhaler core as can be seen, for example, from Fig. 4 as well as Figs. 1 and 2 in which the inhaler core I is visible at the inhaling 10 end. The inhaler is approximately 26 mm across in its widest position and is preferably approximately 20 mm deep at its widest position. This provides the relatively flat elongate configuration apparent from Figs. 1 and 2. Recesses 30 are provided in the top and bottom surfaces of the outer housing 3. This makes the inhaler device 1 easier to grip both during the inhaling process and as it is refilled. During inhaling, the user will grip the inhaler by placing their fingers on the top recess and their thumb against the bottom recess.

All of the features described above are disclosed in WO 2019/073204. However, one significant difference in the inhaler is at the refill end 14. At this end, the smooth generally flat nature of the outer housing 3 is preserved, rather than converging into a small circular refill end as in WO 2019/073204. At this end, at least one groove 31 extends axially from the refill end 14.

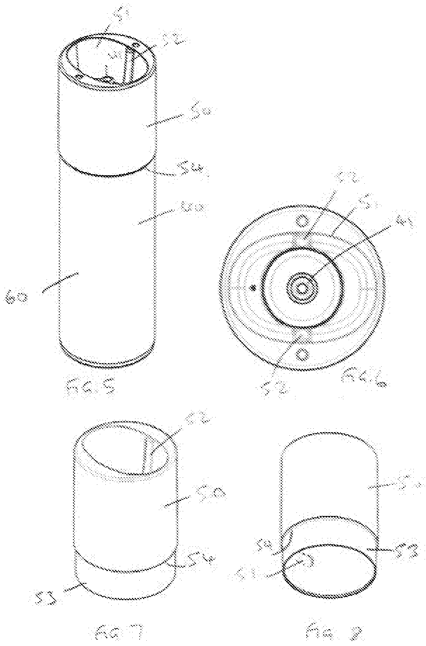

The refill will now be described with reference to Figures 5 to 8.

The refill comprises two components; namely a cylinder 40 and a cap 50. The cylinder is a pressurised refill cylinder of the design which is well known in the art. The cylinder has an outlet valve at the top end and an outlet nozzle 41 via which the composition is dispensed when the outlet nozzle is depressed. The cylinder is a pressurised reservoir which is preferably pressurised by the composition comprising a propellant. The outlet valve may be a non-metered outlet valve, preferably of the type described in the above references related to the refill.

The cap 50 is provided with at least one lug 51 via which the cap is snap fitted onto a shoulder (not shown) at the top of the cylinder. The assembly of the cylinder 40 and cap 50 is then enclosed fully or partially by a sticker 60, which further holds the two components together and can be printed with essential product information. The cap 50 cannot then be removed without first removing the sticker 60.

As show in Figures 5 and 6, the cap 50 has internal passageway 51 extending fully through the cap such that it allows access to the valve stem 41 from above. The passageway 51 has a generally oval shape with a pair of inwardly extending ribs 52 which are complimentary to the grooves 31 on the inhaler.

The lower end of the cap has a reduced diameter portion 53 defining a ridge 54 at its top end. When the cap 50 is on the cylinder 40, the sticker 60 is applied to the cylinder 40 and the reduced diameter portion 53 abutting the ridge 54 to secure the two together and provide a smooth outer surface for the assembly as shown in Fig. 5.

In order to refill the inhaler, the refill end 14 of the inhaler 1 is inserted into the cap 50. The inhaling end 10 is shaped such that it cannot be inserted into the cap 50. The inhaler 1 and refill have a narrow elongate configuration in the direction of engagement of the two components. The refill operation is therefore very stable. This is assisted by the fact that there is a large engagement area between two components caused by the relatively large size of the passage 51 and the fact that the two components cannot rotate with respect to one another. The exact manner by which the refill valve in the inhaler 1 interacts with the valve stem 41 is set out in the above references which provide details of the refill pack.

While certain claims specify a cannabinoid inhaler and require an inhalable composition comprising a cannabinoid, all aspects of the invention can be used with any inhalable pharmaceutical composition.

## Claims

1. A refill for an inhaler (1), the refill comprising a cylinder (40) with an axis and containing a pressurised inhalable composition, and an outlet valve at one end, the outlet valve having a hollow valve stem (41) extending axially from the cylinder; **characterised by** a cap (50) fitted onto the cylinder at the outlet end, the cap extending axially beyond the valve stem, the cap having an internal passage (51) extending through the cap to allow access to the valve stem (41), the internal passage providing a guide wall to guide the inhaler (1), in use, onto the stem (41) during the refill process.

2. A refill according to claim 1, wherein the cap (50) is a snap fit onto the cylinder (40).

3. A refill according to claim 2, wherein a film is applied across the top of the cap (50) to cover the internal passage.

4. A refill according to any preceding claim further comprising a sticker (60) surrounding both the cap (50) and the cylinder (40).

5. A refill according to any preceding claim, wherein the internal passage (51) extending through the cap (50) has a non-circular cross-section.

6. A refill according to claim 5, wherein the internal passage (51) preferably has an elongate shape.

7. A refill according to claim 5 or claim 6, wherein the internal passage (51) has at least one inwardly protecting axially extending rib (52) to provide a location feature with a corresponding recess on the refill end of the inhaler (1).

8. A refill according to any preceding claim, wherein the cross sectional area of the internal passage (51) in a plane perpendicular to the axial direction is at least 30% of the cross sectional area of the cap (50) in a plane perpendicular to the axial direction.

9. A refill according to any preceding claim, wherein the cross sectional area of the internal passage (51) in a plane perpendicular to the axial direction is at least 40% of the cross sectional area of the cap (50) in a plane perpendicular to the axial direction.

10. A refill according to any preceding claim, wherein the valve stem (41) is depressible axially to open the valve.

11. A refill according to any one of the preceding claims in combination with an inhaler (1), the inhaler having a housing (3) with a refill end (14) and an inhaling end (10), the refill end being dimensioned to fit within the internal passage (51) in the refill, and the inhaling end being dimensioned so that it will not fit within the internal passage in the refill.

## Patentansprüche

1. Nachfüllpackung für einen Inhalator (1), wobei die Nachfüllpackung einen Zylinder (40) mit einer Achse, der eine unter Druck stehende inhalierbare Zusammensetzung enthält, und ein Auslassventil an einem Ende umfasst, wobei das Auslassventil einen hohlen Ventilschaft (41) aufweist, der sich axial von dem Zylinder erstreckt; **gekennzeichnet durch** eine Kappe (50), die auf den Zylinder am Auslassende aufgesetzt ist, wobei sich die Kappe axial über den Ventilschaft hinaus erstreckt, wobei die Kappe einen inneren Durchgang (51) aufweist, der sich durch die Kappe hindurch erstreckt, um den Zugang zum Ventilschaft (41) zu ermöglichen, wobei der innere Durchgang eine Führungswand bereitstellt, um den Inhalator (1) beim Gebrauch während des Nachfüllvorgangs auf den Schaft (41) zu führen.

2. Nachfüllvorrichtung nach Anspruch 1, bei der die Kappe (50) auf dem Zylinder (40) eingerastet ist.

3. Nachfüllpackung nach Anspruch 2, bei der eine Folie über der Oberseite der Kappe (50) angebracht ist, um den inneren Durchgang abzudecken.

4. Nachfüllpackung nach einem der vorhergehenden Ansprüche, ferner mit einem Aufkleber (60), der sowohl die Kappe (50) als auch den Zylinder (40) umgibt.

5. Nachfüllpackung nach einem der vorhergehenden Ansprüche, bei der der innere Durchgang (51), der sich durch die Kappe (50) erstreckt, einen nicht kreisförmigen Querschnitt aufweist.

6. Nachfüllpackung nach Anspruch 5, bei der der innere Durchgang (51) vorzugsweise eine längliche Form aufweist.

7. Nachfüllpackung nach Anspruch 5 oder 6, bei der der innere Durchgang (51) mindestens eine nach innen schützende, sich axial erstreckende Rippe (52) aufweist, um ein Positionierungsmerkmal mit einer entsprechenden Ausnehmung am Nachfüllende des Inhalators (1) bereitzustellen.

8. Nachfüllpackung nach einem der vorhergehenden Ansprüche, bei der die Querschnittsfläche des inneren Durchgangs (51) in einer Ebene senkrecht zur axialen Richtung mindestens 30% der Querschnittsfläche der Kappe (50) in einer Ebene senkrecht zur axialen Richtung beträgt.

9. Nachfüllpackung nach einem der vorhergehenden Ansprüche, bei der die Querschnittsfläche des inneren Durchgangs (51) in einer Ebene senkrecht zur axialen Richtung mindestens 40 % der Querschnittsfläche der Kappe (50) in einer Ebene senkrecht zur axialen Richtung beträgt.

10. Nachfüllvorrichtung nach einem der vorhergehenden Ansprüche, bei der der Ventilschaft (41) axial eindrückbar ist, um das Ventil zu öffnen.

11. Nachfüllpackung nach einem der vorhergehenden Ansprüche in Kombination mit einem Inhalator (1), wobei der Inhalator ein Gehäuse (3) mit einem Nachfüllende (14) und einem Inhalationsende (10) aufweist, wobei das Nachfüllende so dimensioniert ist, dass es in den inneren Durchgang (51) in der Nachfüllpackung passt, und das Inhalationsende so dimensioniert ist, dass es nicht in den inneren Durchgang in der Nachfüllpackung passt.

## Revendications

1. Recharge pour un inhalateur (1), la recharge comprenant un cylindre (40) avec un axe et contenant une composition inhalable sous pression, et une soupape de sortie à une extrémité, la soupape de sortie présentant une tige de soupape creuse (41) s'étendant axialement à partir du cylindre ; **caractérisée par** un capuchon (50) ajusté sur le cylindre au niveau de l'extrémité de sortie, le capuchon s'étendant axialement au-delà de la tige de soupape, le capuchon présentant un passage interne (51) s'étendant à travers le capuchon pour permettre un accès à la tige de soupape (41), le passage interne fournissant une paroi de guidage pour guider l'inhalateur (1), en utilisation, jusque sur la tige (41) pendant le processus de recharge.

2. Recharge selon la revendication 1, dans laquelle le capuchon (50) est ajusté par encliquetage sur le cylindre (40) .

3. Recharge selon la revendication 2, dans laquelle un film est appliqué sur la partie supérieure du capuchon (50) pour couvrir le passage interne.

4. Recharge selon l'une quelconque des revendications précédentes, comprenant en outre un autocollant (60) entourant à la fois le capuchon (50) et le cylindre (40).

5. Recharge selon l'une quelconque des revendications précédentes, dans laquelle le passage interne (51) s'étendant à travers le capuchon (50) présente une section transversale non circulaire.

6. Recharge selon la revendication 5, dans laquelle le passage interne (51) présente de préférence une forme allongée.

7. Recharge selon la revendication 5 ou la revendication 6, dans laquelle le passage interne (51) présente au moins une nervure (52) s'étendant axialement de protection vers l'intérieur pour fournir une caractéristique de localisation avec un évidement correspondant sur l'extrémité de recharge de l'inhalateur (1).

8. Recharge selon l'une quelconque des revendications précédentes, dans laquelle l'aire de section transversale du passage interne (51) dans un plan perpendiculaire à la direction axiale est d'au moins 30 % de l'aire de section transversale du capuchon (50) dans un plan perpendiculaire à la direction axiale.

9. Recharge selon l'une quelconque des revendications précédentes, dans laquelle l'aire de section transversale du passage interne (51) dans un plan perpendiculaire à la direction axiale est d'au moins 40 % de l'aire de section transversale du capuchon (50) dans un plan perpendiculaire à la direction axiale.

10. Recharge selon l'une quelconque des revendications précédentes, dans laquelle la tige de soupape (41) peut être enfoncée axialement pour ouvrir la soupape.

11. Recharge selon l'une quelconque des revendications précédentes en combinaison avec un inhalateur (1), l'inhalateur présentant un boîtier (3) avec une extrémité de recharge (14) et une extrémité d'inhalation (10), l'extrémité de recharge étant dimensionnée pour s'ajuster à l'intérieur du passage interne (51) dans la recharge, et l'extrémité d'inhalation étant dimensionnée de sorte qu'elle ne s'ajuste pas à l'intérieur du passage interne dans la recharge.
